# FASCICULE DE BREVET EUROPEEN

(11) **EP 0 561 089 B1**
(45) Date de publication et mention de la délivrance du brevet: **05.02.1997**
(21) Numéro de dépôt: 92403560.3
(22) Date de dépôt: 28.12.1992
(51) Int. Cl.: C08B 30/18, C12P 19/20, C12P 19/22, A23L 1/09, A23L 1/236

(54) **Composition de saccharides hydrogénés hypocariogènes, procédé de préparation et application de cette composition**
Zusammensetzung von hydrierten Sacchariden, die die Kariesbildung vermindert, Verfahren zu ihrer Herstellung und ihre Anwendung
Low-cariogenic hydrogenated saccharide composition, process for obtaining the same and its use

(30) Priorité: 19.03.1992 FR 9203314
(43) Date de publication de la demande: 22.09.1993
(73) Titulaire: Roquette Frères, F-62136 Lestrem (FR)
(72) Inventeur: Caboche, Jean-Jacques, F-62400 Bethune (FR)
(74) Mandataire: Boulinguiez, Didier

(56) Documents cités:
- EP-A- 0 094 292
- EP-A- 0 171 964
- EP-A- 0 368 451
- WO-A-85/02758

## Description

L'invention a pour objet une composition de saccharides hydrogénés hypocariogènes utilisable comme composition édulcorante ou comme agent de texture dans les produits destinés à être ingérés par les hommes ou les animaux, c'est-à-dire notamment dans les produits alimentaires, et dans certains produits pharmaceutiques ou vétérinaires.

L'invention vise également un procédé de préparation de ladite composition, ainsi que l'application de cette composition dans les produits destinés à être ingérés par les hommes ou les animaux.

Par l'expression "produits destinés à être ingérés par les hommes ou les animaux", on entend les produits destinés à l'ingestion et à l'administration orale tels que diverses denrées alimentaires comme les confiseries, les pâtisseries, les crèmes, les boissons, les confitures, les sauces, les crèmes glacées, les aliments préparés pour animaux, ainsi que les produits pharmaceutiques, vétérinaires, diététiques ou hygiéniques tels que par exemple les élixirs, les sirops contre la toux, les tablettes ou les comprimés, les pâtes à mâcher, les chewing-gums, les pastilles, les solutions d'hygiène buccale, les pâtes et les gels dentifrice.

Par l'expression "saccharides hydrogénés hypocariogènes", on entend des saccharides hydrogénés qui présentent une moindre acidification par les bactéries de la bouche que les sucres classiques tels que le saccharose, le glucose ou le fructose.

De tels saccharides hydrogénés hypocariogènes sont déjà connus. On peut ainsi citer par exemple le sorbitol, le xylitol, le maltitol, l'érythritol, le lactitol, l'isomaltulose hydrogéné (connu sous la marque PALATINIT ^{R} ou plus généralement sous l'appellation ISOMALT), le mannitol, l'arabitol, le thréitol, l'isomaltitol.

Des sirops contenant certains de ces différents produits sont déjà commercialisés comme par exemple les sirops de sorbitol, les sirops de maltitol à environ 50 - 55 % de maltitol, comme par exemple le LYCASIN ^{R} 80/55 commercialisé par la société demanderesse, ou les sirops de maltitol à environ 72 - 78 % de maltitol sur matière sèche, comme par exemple le MALTISORB^{R} 75/75 commercialisé par la société demanderesse ou les sirops de maltitol vendus sous les marques MALTIDEX^{R} 100, MALTIDEX^{R} 200, MALBIT^{R} et FINMALT^{R}.

Aucun des produits cités précédemment, ou des mélanges de ces différents produits, ne présente cependant toutes les qualités, avantages ou aptitudes technologiques que l'on souhaiterait trouver dans une composition édulcorante hypocariogène.

En effet, certains des produits précités, comme par exemple le sorbitol, le maltitol, le xylitol, l'érythritol ou le mannitol, quand ils sont employés à des richesses et à des concentrations élevées, ont une fâcheuse tendance à cristalliser, ce qui les rend impropres à une utilisation dans nombre de produits alimentaires, pharmaceutiques ou vétérinaires.

D'autre part, certains produits comme le mannitol, le lactitol, le thréitol, l'isomaltulose hydrogéné, ne présentent pas un pouvoir sucrant élevé. Ceci limite leurs applications par exemple dans les articles de confiserie ou les sirops pharmaceutiques où l'on recherche avant toute chose une saveur sucrée prononcée. Ceci nécessite l'adjonction d'édulcorants de synthèse tels que la saccharine, l'aspartame ou les cyclamates et l'acésulfame K, qui sont des produits relativement chers et qui peuvent être instables.

Quelques produits présentent cependant un pouvoir sucrant élevé et sont difficilement cristallisables. C'est le cas par exemple de certains sirops de maltitol. Néanmoins, ces sirops présentent alors le gros désavantage de ne pas conférer suffisamment de viscosité aux articles dans lesquels ils sont utilisés. Or, une telle viscosité est requise dans des articles comme les pâtes à mâcher, les bonbons au sucre cuit, les nougats, les sirops ou les élixirs pharmaceutiques ou les pâtes dentifrice.

Certains des produits hydrogénés précités sont par ailleurs très hygroscopiques, ce qui peut présenter des problèmes notamment dans la fabrication des bonbons au sucre cuit, les bonbons fabriqués présentant alors une tendance certaine à la reprise en eau et des inconvénients de collage au papier d'emballage.

Il existerait d'autre part un intérêt manifeste à pouvoir faire varier l'activité de l'eau des compositions édulcorantes en fonction des utilisations ultérieures visées. Or, cette possibilité n'existe pas à l'heure actuelle, même en utilisant des mélanges de deux ou trois produits hydrogénés tels que ceux qui ont été précités.

Enfin, pour certaines applications, il serait également souhaitable de pouvoir faire varier à volonté la température d'ébullition de la composition édulcorante, de modifier son hygroscopicité, ou encore de modifier dans un sens donné la température de transition vitreuse ou le point de congélation.

Il existe donc une demande tout à fait évidente de l'industrie alimentaire et de l'industrie pharmaceutique ou vétérinaire de pouvoir disposer d'une composition édulcorante
- qui soit hypocariogène, et présente une grande stabilité aux enzymes,
- qui présente un pouvoir sucrant élevé,
- qui ne nécessite pas forcément l'addition d'édulcorants de synthèse (ceci pouvant entraîner des caractéristiques organoleptiques désagréables ou des limitations dans la stabilité à la chaleur),
- qui présente de bonnes aptitudes technologiques dans tous types d'application,
- qui soit compatible avec la très grande majorité des ingrédients utilisés dans les produits destinés à être ingérés et puisse être éventuellement prémélangé à l'un ou l'autre d'entre eux,
- dont l'activité de l'eau puisse être réglée facilement en fonction de l'application envisagée,
- dont l'hygroscopicité, la température de transition vitreuse, le point de congélation, la température d'ébullition puissent être modulés en fonction de l'application choisie,
- qui présente une viscosité et une texture suffisantes afin de conférer aux articles finis des propriétés "mécaniques" et une texture acceptables,
- qui ne nécessite pas l'adjonction d'additifs texturants ou de produits auxiliaires,
- qui ne présente pas de risques de cristallisation dans les applications où un tel risque serait dommageable,
- ou qui puisse au contraire induire des cristallisations limitées et contrôlées dans certaines applications où une microcristallisation ou un grainage de surface sont délibérément recherchés.

Or, la société demanderesse a eu le grand mérite de pouvoir concilier toutes ces nombreuses qualités et propriétés, réputées jusqu'alors inconciliables, en élaborant et en mettant au point une nouvelle composition de saccharides hydrogénés hypocariogènes.

La composition de saccharides hydrogénés hypocariogènes conforme à l'invention est ainsi caractérisée par le fait qu'elle présente, les teneurs étant exprimées en poids par rapport à la matière sèche de ladite composition :
- une teneur de 0,1 à 80 %, de préférence de 0,1 à 75 % et plus préférentiellement encore de 0,1 à 70 % en monosaccharides hydrogénés,
- une teneur de 0,1 à 96 %, de préférence de 0,2 à 94 % et plus préférentiellement encore de 0,3 à 90 % en poids de disaccharides hydrogénés,
- une teneur de 11 % à 96 %, de préférence de 22 % à 94 % et plus préférentiellement de 35 à 90 % en poids de mono et de disaccharides hydrogénés,
- une teneur de 1 à 40 %, de préférence de 1,5 à 30 % et plus préférentiellement encore de 3 à 26,5 % en poids de polysaccharides non hydrolysables par l'amyloglucosidase dans un test F (décrit ci-après),
- le complément à 100 % étant constitué par des oligo et polysaccharides hydrogénés.

Les monosaccharides hydrogénés peuvent être choisis dans le groupe comprenant le sorbitol, le mannitol, le galactitol, le xylitol, le thréitol, l'arabitol et l'érythritol.

Les disaccharides hydrogénés peuvent être choisis dans le groupe comprenant le maltitol, le maltulose hydrogéné, l'isomaltulose hydrogéné (mélange de glucopyranosido-1,6-mannitol et de glucopyranosido-1,6-sorbitol), l'isomaltitol, le lactitol, l'inulobiose hydrogéné.

Les oligosaccharides et polysaccharides hydrogénés peuvent être constitués par le maltotriitol, le maltotétraitol et autres oligo- et poly-saccharides hydrogénés obtenus par hydrolyse de l'amidon suivie d'une hydrogénation. Lesdits oligo-saccharides et poly-saccharides hydrogénés peuvent cependant être également constitués par le cellobiitol, le cellotriitol, le xylobiitol, le xylotriitol, l'inulotriitol et autres oligo- et poly- saccharides hydrogénés obtenus par hydrolyse, généralement acide, de cellulose de xylans, de fructans comme par exemple l'inuline, suivie d'une hydrogénation.

Afin de déterminer sur les compositions la teneur en polysaccharides non hydrolysables par l'enzyme amyloglucosidase, on utilise le test F qui correspond au test de détermination des "fibres alimentaires totales" développé par la société SIGMA Chemical Company P.O. Box 14508, St Louis, M.O. 63178 USA et qui est décrit en détail dans le bulletin technique SIGMA No TDFAB-1 de juin 1991.

Ce test consiste essentiellement à déterminer la quantité de matière contenue dans l'hydrolysat, non hydrolysable par une amyloglucosidase en présence d'une α-amylase thermorésistante et d'une protéase. Cette quantité est exprimée en pourcentage par rapport à une quantité de 1 g environ d'hydrolysat préalablement séché sous vide à 70°C pendant une nuit.

Pour conduire ce test, on procède comme suit :
1) On pèse à 0,1 mg près quatre échantillons de 1 g environ d'hydrolysat préalablement séché sous vide et refroidi dans un dessicateur pendant 1 nuit, que l'on introduit dans un bécher forme haute de 400 ml.
2) On ajoute dans chacun des quatre béchers 50 ml d'un tampon phosphate (0,05 M) à pH : 6,0.
3) On ajoute 0,05 ml d'une solution d'alpha-amylase (produit Sigma n° A 3306) dans chacun des béchers, et l'on mélange intimement.
4) On couvre chaque bécher avec un film aluminium avant de les placer dans un bain d'eau bouillante pour les incuber pendant 30 mn à partir du moment où la température atteint dans les béchers 95°C. On agite doucement à intervalles réguliers de 5 minutes.
5) On refroidit les solutions à température ambiante.
6) On ajuste le pH des solutions à 7,5 ± 0,1 par ajout dans chaque bécher de 10 ml de NaOH 0,171N. On vérifie le pH et on l'ajuste éventuellement avec de la soude (0,171 N) ou de l'acide phosphorique (0,205 M).
7) On ajoute 5 mg de protéase en poudre (produit Sigma n° P-3910) à chacun des béchers.
8) On couvre les béchers avec un film d'aluminium et on les incube à 60°C pendant 30 mn sous agitation continue. Le temps d'incubation de 30 mn débute à partir du moment où la température interne des béchers atteint 60°C.
9) On refroidit à température ambiante.
10) On ajoute 10 ml d'H3PO4 0,205 M à chacun des béchers pour ajuster le pH à 4,5 ± 0,2. On vérifie le pH. On ajuste éventuellement avec précaution avec les solutions de soude ou d'acide phosphorique.
11) On ajoute 0,3 ml d'amyloglucosidase (produit Sigma N A.9913) à chaque bécher.
12) On couvre chacun des béchers avec un film d'aluminium et on incube pendant 30 minutes à 60°C sous agitation continue. Le temps d'incubation de 30 mn débute à partir du moment où la température interne des béchers atteint 60°C.
13) On ajoute 280 ml d'éthanol à 95 % (v/v), préchauffé à 60°C, à chacun des béchers. (éthanol à 95 % v/v : 50 ml d'eau déminéralisée, éthanol absolu q.s.p. 1000 ml à 20°C).
14) On laisse se former un précipité par abandon à température ambiante pendant au moins 60 minutes, ou une nuit (même temps pour chacun des 4 essais).
15) On filtre sous vide, sur creuset de verre fritté et lit de Celite le contenu de chacun des béchers qu'on lave successivement et avec précaution avec :
   - trois fois 20 ml d'éthanol à 78 % (v/v) (éthanol à 78 % v/v : 220 ml d'eau déminéralisée, éthanol absolu q.s.p. 1000 ml à 20°C)
   - deux fois 10 ml d'éthanol à 95 % (v/v)
   - et deux fois 10 ml d'acétone.
16) On sèche les quatre filtres à 70°C sous vide pendant 1 nuit.
17) On refroidit ces filtres dans un dessicateur avant de les peser à 0,1 mg près, considérant ce poids comme la somme du poids de résidu de filtration (polysaccharides non hydrolysables à l'amyloglucosidase, plus protéines plus cendres) et du poids du creuset avec Celite.
18) On détermine les teneurs en protéines de deux des quatre résidus de filtration provenant des quatre essais en procédant selon la méthode Kjeldahl en utilisant le coefficient de correction 6,25.
19) On détermine les quantités de cendres sur les deux autres résidus de filtration en plaçant les creusets dans un four à 525°C pendant 5 heures.
20) On calcule, comme indiqué dans le Bulletin technique SIGMA, les quantités de polysaccharides non hydrolysables à l'amyloglucosidase pour les quatre essais et on réalise une moyenne de ces quantités que l'on ramène à la moyenne des quantités de matière d'hydrolysat séché à 70°C sous vide pendant une nuit, en tenant compte dans le calcul de résultats de quatre essais à blanc (sans hydrolysat sec), menés en parallèle.

Ce test F constitue une variante du test de détermination des fibres alimentaires totales dans les aliments décrits dans "J. Assoc. Off. Anal. Chem." Vol 68, N° 2, 1985, p 399.

Il présente l'avantage d'être standardisé, de pouvoir être effectué à l'aide d'un kit complet d'analyse et d'être répétable et reproductible.

On peut également déterminer sur les compositions conformes à l'invention les teneurs en polysaccharides précipitables dans l'éthanol et non hydrolysables par l'amyoglucosidase par un autre test que l'on dénomme test A, en procédant comme suit.

Un échantillon de 10 g de composition édulcorante conforme à l'invention amenée par addition d'eau ou évaporation à un Brix de 75 ± 0,2, soit un indice de réfraction de 1,478 environ est utilisé pour la détermination du taux de polysaccharides hydrogénés précipitables dans l'éthanol. On rappelle que le Brix est une unité de mesure couramment employée dans l'industrie amidonnière, et que le Brix d'un sirop est déterminé très aisément par lecture au réfractomètre. Un Brix de 75 correspond généralement pour les compositions conformes à l'invention à une matière sèche approximativement égale à 75 %.

L'échantillon de 10 g de composition édulcorante conforme à l'invention à 75 Brix est additionné de 30 cm3 d'eau distillée et de 60 cm3 d'éthanol absolu. Le mélange est laissé reposer pendant une heure à O°C. Il est ensuite centrifugé à O°C pendant 15 mn à 10 000g. Le culot obtenu est séché en étuve sous vide maintenue à 80°C. Le poids de précipité obtenu, P1, représente le poids de polysaccharides précipitables dans l'éthanol contenu dans les 10 g d'échantillon de départ, soit environ 7,5 g de matière sèche.

Afin de déterminer la teneur de la composition édulcorante conforme à l'invention en polysaccharides hydrogénés précipitables dans l'éthanol et non hydrolysables par l'amyloglucosidase, on utilise un test A, qui consiste à faire subir aux polysaccharides précipités dans l'éthanol obtenus précédemment une attaque enzymatique à l'aide d'une alphaamylase thermorésistante, d'une protéase et d'une amyloglucosidase, puis à opérer une précipitation des polysaccharides non hydrolysables avec de l'éthanol à 95 %, à filtrer le précipité ainsi obtenu, à laver ce dernier plusieurs fois à l'alcool et à l'acétone et enfin à déterminer le poids, P2, de résidu obtenu.

Ce test est également décrit dans "J. ASSOC. OF ANAL. CHEM." Vol. 68, N° 2, 1985, P. 399, article auquel on pourra se référer.

Une composition de saccharides hydrogénés hypocariogènes conforme à l'invention peut être caractérisée par le fait qu'elle présente, les teneurs étant exprimées en poids par rapport à la matière sèche de ladite composition :
- une teneur de 0,1 à 65 % de préférence de 0,1 à 60 % et plus préférentiellement encore de 0,1 à 55 % en monosaccharides hydrogénés,
- une teneur de 10 à 96 %, de préférence de 15 à 94 % et plus préférentiellement encore de 15 à 90 % en poids de disaccharides hydrogénés,
- une teneur de 1 à 30 %, de préférence de 1,5 à 25 % et plus préférentiellement encore de 2 à 15 % en poids de polysaccharides précipitables dans l'éthanol et non hydrolysables par l'amyloglucosidase dans un test A décrit ci-avant.

C'est essentiellement grâce à la présence de la teneur sélectionnée en polysaccharides hydrogénés non hydrolysables par l'amyloglucosidase que l'on peut obtenir, en sélectionnant d'autre part de façon concomitante la teneur en mono-, en di-, en oligo- et en poly- saccharides hydrogénés, une composition édulcorante présentant toutes les qualités recherchées dans la majorité des applications comme par exemple l'absence de cristallisation, la possibilité d'ajuster la viscosité, la température d'ébullition, la température de transition vitreuse, le point de congélation, l'hygroscopicité et le pouvoir sucrant.

Pour préparer la composition édulcorante conforme à l'invention, on s'y prend comme suit ou d'une manière équivalente.

On prépare tout d'abord une fraction comprenant les polysaccharides non digestibles à l'amyloglucosidase. Pour ce faire, on soumet au moins une dextrine et/ou un polyglucose à un traitement enzymatique comportant au moins l'action d'une enzyme saccharifiante telle que l'amyloglucosidase ou la bétamylase, les conditions de ce traitement étant choisies de façon telle que le DE de l'hydrolysat de dextrine et/ou de polyglucose obtenu soit compris entre 5 et 80, de préférence entre 10 et 65, à l'issue de ce traitement, l'hydrolysat obtenu étant ensuite hydrogéné puis purifié de manière connue en soi.

Par le terme "polyglucose", on entend les produits composés majoritairement de liaisons 1-6, obtenus par condensation ou réarrangement, à partir du glucose ou d'un ou plusieurs sucres éventuellement réduits, sous l'action combinée de la chaleur et d'acides dans un milieu presque dépourvu d'eau. De tels polymères ont été décrits de nombreuses fois et peuvent être obtenus par des procédés tels que ceux décrits notamment dans les brevets US 2 436 967, US 3 766 165, US 4 965 354, US 5 051 500, JP 01-12761 et JP 02-163101. De façon avantageuse, ces polymères sont obtenus à partir de glucose et d'acide citrique, éventuellement en présence de sorbitol.

Dans le cadre de la présente invention on entend par le terme "dextrine" les produits obtenus par chauffage de l'amidon amené à un faible taux d'humidité, en présence généralement de catalyseurs acides ou basiques. Ce "grillage à sec" de l'amidon, le plus couramment en présence d'acide, entraîne à la fois une dépolymérisation de l'amidon et une condensation des fragments d'amidon obtenus, et conduit à l'obtention de molécules très ramifiées. Les dextrines font partie des dérivés d'amidon les plus anciens et leur préparation, leurs applications, les différents types de dextrines ainsi que leurs propriétés sont décrits par exemple dans l'ouvrage intitulé "Starch Chemistry and Technology" - Second Edition - Edited by Roy L. WHISTLER - 1984 - Academic Press Inc.

Des traitements enzymatiques de dextrine ont déjà été proposés dans la littérature, comme par exemple dans les demandes de brevet européen n° 0368451 ou JP-A-62091501, mais pour d'autres buts. Ces documents de l'art antérieur seront plus amplement analysés par la suite.

De préférence, on utilise pour la préparation des compositions conformes à l'invention des dextrines obtenues par grillage à sec de l'amidon en présence d'un catalyseur acide comme l'acide chlorhydrique. L'acide est ainsi pulvérisé sur l'amidon et le mélange obtenu est préséché, par exemple de 80 à 130°C jusqu'à une teneur en eau inférieure ou égale à environ 5 %. Puis le mélange est "grillé" à une température d'environ 140° à 250°C pendant une durée de 30 minutes à environ 6 heures pour obtenir la dextrine, qui présente en fin de réaction un DE de 0,5 à 10 environ. On peut utiliser, pour la préparation de ces dextrines, n'importe quel type d'amidon, et notamment l'amidon de maïs, l'amidon de pomme de terre, l'amidon de blé, l'amidon de manioc, l'amidon de riz ou l'amidon de pois.

D'après la norme ISO 1227 de 1979, une dextrine est obtenue à partir d'amidon ou de fécule transformés par chauffage à sec avec ou sans addition de petites quantités de réactifs chimiques. Traditionnellement, les dextrines sont classées en deux catégories : les dextrines blanches dont l'aspect est peu différent de celui de la matière première utilisée, et les dextrines jaunes, produites dans des conditions plus drastiques et dont l'intensité de la coloration peut être corrélée au degré de modification de la structure native. Les quatre types de réaction intervenant lors de la dextrinification sont, aux faibles températures, essentiellement l'hydrolyse des liaisons alpha 1-4 puis, aux températures plus élevées, des réactions de condensation, de transglycosidation et d'anhydrisation.

Des dextrines telles que celles commercialisées par la société demanderesse sous les marque TACKIDEX DF 165, TACKIDEX DF 155, TACKIDEX JO 55 K peuvent être avantageusement utilisées.

La dextrine, le polyglucose ou le mélange de dextrines et/ou de polyglucoses sélectionnés, sont ainsi mis en suspension dans l'eau à une matière sèche d'environ 20 à 70 %, de préférence de 20 à 45 % afin de subir la saccharification à l'aide d'au moins une enzyme saccharifiante constituée par l'amyloglucosidase et/ou la bétaamylase.

De préférence, et bien que cela ne soit pas nécessaire dans tous les cas, on peut faire précéder cette action enzymatique de la bétaamylase et/ou de l'amyloglucosidase par l'action d'une alphaamylase, de préférence thermorésistante.

De même, on peut faire suivre ou accompagner le traitement de saccharification par l'action d'une alphaamylase.

Selon un mode préféré, on peut également faire appel à un traitement à l'aide d'une enzyme hydrolysant les liaisons 1-6 de l'amylopectine telle que par exemple l'isoamylase ou la pullulanase. Ce traitement à l'aide d'une enzyme hydrolysant les liaisons 1-6 de l'amylopectine qui peut précéder, accompagner ou suivre le traitement de saccharification est particulièrement avantageux dans la mesure où l'action de cette enzyme permet de ne laisser subsister dans l'hydrolysat de dextrine et/ou de polyglucose obtenu que les polysaccharides très difficilement digestibles enzymatiquement.

L'action enzymatique de l'amyloglucosidase, de la bétaamylase et éventuellement de l'alphaamylase, de la pullulanase ou de l'isoamylase, sur la dextrine, le polyglucose ou sur leur mélange, permet d'obtenir une fraction comportant à côté de glucose, de maltose, de maltotriose et autres oligosaccharides et polysaccharides des polysaccharides non digestibles par l'amyloglucosidase.

Cette fraction peut être directement hydrogénée, ou subir un traitement supplémentaire en vue de l'enrichir en polysaccharides non digestibles par l'amyloglucosidase. Un tel traitement peut consister par exemple en une opération sur membrane comme l'osmose inverse ou l'ultrafiltration, de précipitation par solvants ou de fractionnement chromatographique. On peut même, si on le désire, aller jusqu'à pratiquement isoler lesdits polysaccharides, de préférence par fractionnement chromatographique sur résines cationiques sous forme alcaline ou alcalino-terreuse ou sur zéolites.

Ce fractionnement chromatographique peut donc être réalisé avant hydrogénation et il y a bien évidemment lieu alors d'hydrogéner par la suite les polysaccharides non digestibles par l'amyloglucosidase. Mais conformément à une réalisation préférentielle de l'invention, ce traitement d'enrichissement ou de séparation chromatographique des polysaccharides est effectué après hydrogénation. Ceci permet ainsi de ne recourir qu'à un seul traitement d'hydrogénation pour la préparation de la composition de saccharides hydrogénés conforme à l'invention, et d'autre part la séparation des polysaccharides est dans ce cas plus efficace.

Ayant donc ainsi obtenu une fraction contenant des polysaccharides éventuellement hydrogénés non hydrolysables par l'amyloglucosidase, on prépare ensuite la composition édulcorante conforme à l'invention.

Pour ce faire, partant d'un pourcentage choisi de cette fraction, ce pourcentage dépendant bien évidemment de la richesse en polysaccharides de ladite fraction, on ajoute alors des monosaccharides, des disaccharides, des oligosaccharides et polysaccharides éventuellement hydrogénés, dans des proportions choisies en chacun de ces groupes de constituants.

Selon une première variante, on ajoute à la fraction de polysaccharides non hydrogénés obtenue à l'issue du traitement enzymatique de la dextrine, du polyglucose ou du mélange, suivi éventuellement d'une étape d'enrichissement ou de fractionnement chromatographique, des monosaccharides, des disaccharides ou des oligosaccharides et polysaccharides non hydrogénés dans des proportions choisies en chacun de ces différents constituants. Et on réalise l'hydrogénation de la composition ainsi obtenue.

Selon une seconde variante, qui est préférée, on hydrogéne séparément la fraction de polysaccharides, et on y ajoute des monosaccharides ou des disaccharides ou des oligosaccharides et polysaccharides déjà hydrogénés dans des proportions choisies en chacun de ces différents constituants.

A titre d'exemple, on peut ainsi réaliser une composition édulcorante conforme à l'invention en procédant au mélange d'un hydrolysat d'inuline, de bois de bouleau ou de rafles de maïs, d'un hydrolysat d'amidon, et d'une fraction contenant les polysaccharides obtenus par hydrolyse enzymatique d'une dextrine et/ou d'un polyglucose, ces hydrolysats et cette fraction étant mélangés dans des proportions prédéterminées et on réalise ensuite l'hydrogénation du mélange ainsi obtenu.

La composition édulcorante préparée dans ces conditions contient à la fois du xylitol, de l'arabitol, du sorbitol, du mannitol, du maltitol ainsi que des polysaccharides hydrogénés non digestibles par l'amyloglucosidase.

Comme on l'a déjà mentionné précédemment, des procédés consistant à hydrolyser enzymatiquement une dextrine ont déjà été proposés dans la littérature.

Ainsi, la demande de brevet européen n° 0368451 décrit un procédé consistant essentiellement à dissoudre une pyrodextrine dans l'eau et à faire agir une alphaamylase sur la solution résultante. Ce procédé a pour but de retirer l'odeur et le goût indésirables de la pyrodextrine et de conduire à des dextrines contenant des fibres diététiques.

Ainsi selon le procédé décrit dans cette demande de brevet, une pyrodextrine est dissoute dans l'eau puis hydrolysée avec de l'alphaamylase. D'autres enzymes peuvent cependant être ajoutées après le traitement à l'alphaamylase : c'est le cas de la transglucosidase, de la bétaamylase, de la glucoamylase. Le produit obtenu à l'issue de ce traitement enzymatique peut alors être hydrogéné. Des monosaccharides et des oligosaccharides peuvent être ajoutés à l'amidon de départ destiné à être dextrinifié, afin d'augmenter la teneur du produit final en dextrine indigestible.

L'objet de la susdite demande de brevet est donc essentiellement de fournir un produit peu digestible, "à basses calories", agissant comme fibre alimentaire diététique, composé essentiellement de polysaccharides peu digestibles. Le contenu du produit exemplifié en "dextrines indigestibles" varie ainsi entre environ 27 % et environ 95 %, et la teneur de ces produits en polysaccharides de degré de polymérisation égal ou supérieur à 4 est comprise entre environ 60 % et environ 92 % en poids.

Un autre procédé d'obtention de fibres alimentaires diététiques à partir de dextrines a par ailleurs été décrit dans la demande de brevet japonais JP-62091501. Ce procédé consiste à traiter à chaud un hydrolysat d'amidon hydrogéné en opérant ce chauffage dans des conditions anhydres à 150-250°C en présence d'un catalyseur constitué par un acide minéral ou un acide organique.

Tout comme la demande de brevet citée antérieurement, ce document vise donc l'obtention de produits peu digestibles par l'organisme, dits "à basses calories" et agissant dans l'organisme en tant que fibres alimentaires. Leur objet est donc très éloigné de l'objet de la présente invention, qui est de préparer un produit édulcorant constitué de saccharides hydrogénés à haut pouvoir sucrant, hypocariogènes, aux aptitudes technologiques utilisables aussi bien dans les bonbons, les chewing-gums et les pâtes dentifrice que dans les boissons et les élixirs pharmaceutiques ou vétérinaires, ou autres produits.

D'autre part, on peut souligner que lesdits documents de l'art antérieur ne décrivent, ni ne suggèrent, l'utilisation d'enzymes hydrolysant les liaisons 1-6 de l'amylopectine, enzymes dont l'action prévue dans le cadre de la présente invention permet de conduire à une fraction contenant des polysaccharides hydrogénés très peu digestibles, ce qui constitue un avantage tout à fait significatif.

De préférence, les quantités et les conditions d'action des différentes enzymes utilisées pour la préparation de la fraction obtenue par hydrolyse enzymatique de la dextrine et/ou du polyglucose, des dextrines ou des polyglucoses, et destinée à l'élaboration de la composition édulcorante hypocariogène conforme à l'invention, sont choisies parmi les suivantes :
. amyloglucosidase : 4 000 à 500 000 unités internationales/kg de substrat sec, température de 50°C à 60°C, durée d'action de 30 à 100 heures, pH de 5.0 à 6.0,
. bétaamylase : 100 à 10 000 unités LINTNER par kilo de substrat sec, température de 50°C à 60°C, durée d'action de 30 à 100 heures, pH de 5.0 à 6.0,
. alphaamylase : 20 à 2 000 unités KNU (Kilo Novo Units) par kilo de substrat sec, pH de 5.0 à 6.0, température de 50°C à 60°C, durée d'action de 16 à 100 heures,
. enzyme hydrolysant les liaisons 1-6 : 150 à 15 000 unités ABM (ABM, CHESHIRE, ENGLAND) par kilo de substrat sec, éventuellement en présence de 50 à 100 unités internationales de bétaamylase par kilo de substrat sec, pH 5.0 à 6.0, température de 50°C à 60°C, durée d'action de 24 à 100 heures.

Les enzymes mises en oeuvre peuvent être :
. en ce qui concerne l'amyloglucosidase, les amyloglucosidases fongiques,
. en ce qui concerne la bétaamylase, des bétaamylases microbiennes ou végétales,
. en ce qui l'alphaamylase, les alphaamylases bactériennes ou fongiques,
. en ce qui concerne les enzymes hydrolysant les liaisons 1-6, celles choisies parmi la pullulanase et l'isoamylase comme par exemple : PULLUZYME 750 L d'ABM ou CK20L d'AMANO.

Dans le cadre de la présente invention, l'hydrogénation de l'hydrolysat obtenu à la suite de l'hydrolyse enzymatique de la dextrine, du polyglucose ou du mélange de dextrines et/ou de polyglucoses, comprenant éventuellement les saccharides, oligosaccharides et polysaccharides additionnels, peut être effectuée de manière connue en soi par hydrogénation sur nickel de RANEY ou par hydrogénation sur métaux nobles.

Cette hydrogénation est effectuée après purification de l'hydrolysat, par exemple par traitement sur charbon actif, après déminéralisation sur résines cationiques et anioniques. L'hydrogénation peut être effectuée par exemple sur catalyseur au nickel de RANEY, à une température de 130°C et sous une pression d'hydrogène de 50 bars.

Après hydrogénation, le sirop obtenu est filtré puis déminéralisé, puis concentré jusqu'à sa concentration de commercialisation, qui est généralement comprise entre 70 et 85 Brix environ, soit environ 70 % à 85 % de matière sèche. Il peut cependant être également séché, par exemple par atomisation.

L'hydrogénation est généralement conduite jusqu'à l'obtention d'un pourcentage de sucres réducteurs résiduels sur matière sèche inférieur à 0,50, de préférence inférieur à 0,25 et plus préférentiellement encore inférieur à 0,20.

L'une des caractéristiques fondamentales de la composition édulcorante selon l'invention réside dans son hypocariogénicité, c'est-à-dire dans sa propriété de provoquer une bien moindre acidification par les bactéries de la bouche que les sucres conventionnels classiques tels que le glucose, le fructose, le saccharose ou les sirops de glucose.

Selon une réalisation tout à fait avantageuse de l'invention, la composition édulcorante conforme à l'invention présente la propriété de pouvoir être qualifiée de non cariogène selon un test B.

Ce test B avait été mis au point par la société demanderesse afin de contrôler le caractère non cariogène des hydrolysats hydrogénés commercialisés à partir de 1978 sous la dénomination LYCASIN ^{R} 80/55. Ce test simple repose sur la détermination in vitro de l'acidification d'une quantité donnée d'hydrolysat d'amidon hydrogéné après ensemencement du milieu par de la salive. Il repose sur l'appréciation de la chute du pH au cours du temps d'un bouillon de culture contenant le produit à tester, après donc ensemencement avec de la salive provenant de plusieurs donneurs, comparativement à un bouillon de culture témoin ne contenant aucun glucide. Il faut souligner que ce test n'est pas suffisant pour caractériser de façon absolue la non cariogénécité d'un produit, car ses résultats peuvent varier, par exemple, suivant la qualité de la salive utilisée, mais il permet néanmoins d'établir des comparaisons valables entre les différents produits.

Le mode opératoire détaillé de ce test est le suivant.

On prépare une série de tubes contenant 10ml d'un milieu de culture nutritif (milieu trypticase à 2 % de matière sèches) sans sucre à pH 7, et on stérilise ces tubes par passage à l'autoclave à 120°C durant 20 minutes.

Dans une première série de cinq tubes, on introduit 1 ml d'eau stérile pour faire une série témoin.

Dans une deuxième série de cinq tubes, on introduit 1 ml d'une solution à 18% (P/V) du produit à tester.

Puis on ensemence les cinq tubes de chaque série avec un même volume de 0,2ml par tube d'une dilution de salive humaine obtenue par prélèvement sur cinq donneurs.

On suit alors la formation d'acides par mesure du pH, une première mesure étant effectuée avant incubation et les autres mesures étant effectuées après des incubations à 30°C de respectivement 3, 6, 13, 18 et 21 heures.

Pour qu'un produit puisse être considéré comme non cariogène au sens de ce test B, il faut que la différence de pH observée entre le témoin au bout de 21 heures et le produit à tester au bout de 21 heures, ne soit pas trop prononcée et, dans la pratique, au plus égale à 1 unité de pH.

L'un des grands mérites de la présente invention est de fournir des compositions édulcorantes qui présentent la propriété d'être non cariogènes au sens de ce test B, alors qu'elles contiennent pourtant une quantité non négligeable d'oligosaccharides et de polysaccharides hydrogénés.

D'autres mérites de la présente invention sont de fournir des compositions édulcorantes qui soient stables, qui puissent être utilisées en tant qu'agent édulcorant et agent de texture dans les produits destinés à être ingérés par les hommes et les animaux, ces produits présentant une texture liquide ou visqueuse, pâteuse, gélifiée ou solide, qui soient totalement compatibles avec les autres ingrédients utilisés dans ces produits et qui puissent être éventuellement prémélangés sans aucun inconvénient à un conservateur, un émulsifiant, un arôme, un sucre, un polyol, un édulcorant intense, un acide, un principe pharmaceutique ou vétérinaire, une matière grasse, un agent de charge minéral ou organique tel les polydextroses, les fibres, les fructooligosaccharides, les gommes, un agent gélifiant organique ou minéral tel que les protéines, les pectines, les celluloses modifiées, les extraits d'algues et de graines, les polysaccharides bactériens, les silices.

Ces produits destinés à être ingérés par l'homme ou les animaux peuvent présenter une texture liquide ou visqueuse, tels les boissons, les sirops, les émulsions, les suspensions, les élixirs, les bains de bouche, les ampoules buvables ; une texture pâteuse, tels les produits de confiserie non cristallisés ou semi-cristallisés comme les bonbons durs, les gelées, les gommes, les pâtes à macher, les caramels, les chewing gums, les fourrages, les barres céréalières ; une texture gélifiée, tels les gels alimentaires, comme les flans, les confitures, les gelées, les desserts lactés, ou les gels pharmaceutiques et vétérinaires, les dentifrices ; une texture solide, tels les produits de pâtisserie, de biscuiterie, de panification, les comprimés, les entremets, les poudres atomisées ou extrudées d'édulcorant ou d'arômes, les lyophilisats pharmaceutiques ou vétérinaires.

Un autre avantage de la composition de saccharides hydrogénés selon l'invention est d'être particulièrement stable vis-à-vis des enzymes de microorganismes et vis-à-vis des réactifs chimiques, oxydants ou réducteurs.

Cet avantage peut être mis à profit dans la formulation et l'élaboration de produits non destinés à être ingérés par les hommes et les animaux, comme par exemple dans les produits cosmétiques, les plastiques, la trempe des métaux, le traitement des peaux, les moules de fonderie.

Les exemples qui suivent, et qui sont donnés à titre non limitatif, illustreront mieux l'invention.

### EXEMPLE N° 1

Dans une cuve de 25 litres, agitée et thermostatée, on a introduit 20 litres d'un sirop formé par dissolution dans l'eau de dextrine jaune TACKIDEX DF 165 à une matière sèche de 35 %.

Le pH a été ajusté à 5,5 à l'aide de soude concentrée et on a thermostaté la cuve à 55°C avant d'y ajouter :
- 0,15 ₀/₀₀ de bétaamylase SPEZYME DBA, de la Société GENENCOR,
- 2 ₀/₀₀ de pullulanase PULLUZYME 750 L de la Société ABM.

Après 48 heures de saccharification, on a rajouté 1 ₀/₀₀ d'alphaamylase MAXAMYL HT 3000 de la Société GIST-BROCADES. On a stoppé la saccharification au terme de 88 heures.

On a ensuite traité ce sirop par 8 ₀/₀₀ (volume/volume) d'une solution d'H2O2 à 35 % (v/v) pendant 24 heures à 70°C et pH 9,5. L'eau oxygénée résiduelle a été décomposée par addition d'un peu de catalase, puis le sirop a été désoxygéné sous vide avant d'être traité par du charbon actif, puis déminéralisé sur un lit de résines mélangées.

Le sirop a alors été concentré à une matière sèche de 40 % puis a été hydrogéné à l'aide de 5 % de catalyseur au nickel de RANEY, à une température de 130°C et sous une pression d'hydrogène de 50 bars. L'hydrogénation a été poursuivie jusqu'à obtention d'un taux de sucres réducteurs inférieur à 0,5 %.

### Composition A

Une partie de ce sirop conforme à l'invention, appelé par la suite "sirop de base" titrant 15,3 % de maltitol, 22,4 % de polysaccharides de DP égal ou supérieur à 20, et 16,2 % de polysaccharides non hydrolysables à l'amyloglucosidase selon le test F, a alors été additionnée de maltitol cristallisé en proportion telle que le maltitol ajouté représente 40 % de la matière sèche de la composition totale, pour constituer la composition A de saccharides hydrogénés selon l'invention.

### Composition B

Une autre partie de ce sirop de base a été additionnée de xylitol cristallisé en une proportion telle que le xylitol ajouté représente 60 % de la matière sèche de la composition totale, pour constituer la composition B de saccharides hydrogénés selon l'invention.

Les compositions A et B de saccharides hydrogénés selon l'invention ont alors été concentrées à une matière sèche de 75 %.

Ces compositions ont révélé les spectres glucidiques suivants, à côté desquels on a fait figurer les résultats obtenus dans le test F et le test A (tableau I).

**TABLEAU I**

| | Composition A | Composition B |
|---|---|---|
| Matière sèche % | 75 | 75 |
| Sucres réducteurs (% sur MS) | 0,24 | 0,20 |
| DP1 sorbitol | 2,5 | 1,7 |
| DP1 xylitol | 0 | 60 |
| DP2 maltitol et isomaltitol | 48,9 | 6,1 |
| DP3 | 4,4 | 2,6 |
| DP4 | 2,5 | 1,8 |
| DP5 | 3,0 | 1,9 |
| DP6 | 2,5 | 1,9 |
| DP7 | 2,7 | 1,8 |
| DP8 | 2,6 | 1,4 |
| DP 9-20 | 15,5 | 11,1 |
| DP > 20 | 13,8 | 9,0 |
| Polysaccharides non hydrolysables à l'amyloglucosidase en % (test F) | 8,7 | 5,7 |
| Précipité P1 dans l'éthanol (%) (test A) | 23 | 15,3 |
| Précipité P2 (en %) après digestion enzymatique (test A) | 4,6 | 3,1 |
| Viscosité à 20°C (cps) | 7000 | 3200 |

On a effectué sur les compositions A et B un test de cariogénicité selon le test B. La chute de pH entre le témoin et les compositions A et B analysées était respectivement de 0.90 et 0.80. Les compositions A et B ne sont pas cariogènes selon le test B.

### EXEMPLE N° 2

On a préparé un autre sirop de base en s'y prenant comme décrit ci-dessous :

Dans une cuve de 25 litres, agitée et thermostatée, on a introduit 20 litres d'un sirop formé par dissolution dans l'eau de dextrine jaune TACKIDEX DF 165 à une matière sèche de 35 %.

Le pH a été ajusté à 5,5 et la température à 55°C. On y a ajouté 0,05 % d'amyloglucosidase OPTIDEX C 300 de la Société MILES par kg de dextrine et 1 ₀/₀₀ d'alphaamylase MAXAMYL HT 3000 de la Société GIST-BROCADES, puis on a laissé la saccharification se dérouler durant 60 heures.

Le sirop obtenu a été traité à l'eau oxygénée comme il a été décrit dans l'exemple 1 avant d'être traité sur charbon actif puis déminéralisé sur un lit de résines mélangées. On l'a ensuite concentré à une matière sèche de 50 %. Ce sirop contenait 45 % de glucose vrai et 55 % d'oligo et polysaccharides.

On a fractionné ce sirop par chromatographie sur une colonne de résine cationique forte réticulée au divinylbenzène et permutée sous la forme sodium, d'une granulométrie comprise entre 0,2 et 0,4 mm : résine C204, commercialisée par DUOLITE. Le fractionnement a été effectué par charges successives de 0,15 litre de sirop pour une colonne d'une hauteur de 2 mètres contenant 1,50 litre de résine . L'élution de chaque charge a été réalisée par de l'eau à 60°C, à un débit de 0,85 litre/heure.

Chaque charge d'éluat a été fractionnée en deux parties, l'une correspondant au début d'élution et contenant les polysaccharides, l'autre correspondant à la fin de l'élution et contenant essentiellement du glucose.

Les éluats correspondant aux fractions riches en polysaccharides ont été évaporés puis reconcentrés pour fournir un sirop de polysaccharides dont le spectre glucidique, déterminé par chromatographie liquide à haute pression, est présenté dans le tableau II.

**TABLEAU II**

| | |
|---|---|
| DP 1 (glucose) | 0,4 % |
| DP 2 (maltose, isomaltose) | 0,8 % |
| DP 3 (maltotriose) | 2,5 % |
| DP 4 (maltotétraose) | 4,0 % |
| DP 5 | 5,4 % |
| DP 6 | 4,7 % |
| DP 7 | 3,9 % |
| DP 8 | 4,2 % |
| DP 9-20 | 31,6 % |
| DP > 20 | 42,6 % |

Ce sirop, appelé par la suite sirop de polysaccharides, a servi de base pour confectionner les compositions suivantes C et D selon l'invention.

### Composition C

Du xylose cristallisé a été dissous dans le sirop de polysaccharides de façon à représenter 50 % de la matière sèche de la composition totale, puis le sirop résultant a été hydrogéné, purifié et concentré de façon classique.

### Composition D

Du xylose cristallisé a été dissous dans le même sirop de polysaccharides de façon à représenter 66 % de la matière sèche, puis le sirop résultant a été hydrogéné, purifié et concentré de la même façon classique.

On a donc obtenu des sirops dont la composition, sur matière sèche, est présentée dans le tableau III.

**TABLEAU III**

| | Composition C | Composition D |
|---|---|---|
| Matière sèche % | 75 | 75 |
| Monosaccharides hydrogénés (xylitol + sorbitol) | 50,2 | 66,1 |
| Maltitol-isomaltitol | 0,4 | 0,3 |
| DP3 | 1,25 | 0,8 |
| DP4 | 2 | 1,5 |
| DP5 | 2,7 | 1,8 |
| DP6 | 2,35 | 1,6 |
| DP7 | 1,95 | 1,3 |
| DP8 | 2,1 | 1,4 |
| DP 9-20 | 15,8 | 10,5 |
| DP >20 | 21,3 | 14,2 |
| Polysaccharides non hydrolysables en % (Test F) | 23,2 | 15,8 |
| Précipité P1 (%) (Test A) | 28,1 | 18,7 |
| Précipité P2 (%) (Test A) | 21,6 | 14,4 |
| Viscosité (cps) | 5900 | 4800 |

### EXEMPLE N° 3

Le sirop de polysaccharides obtenu selon l'exemple 2 et dont la composition est donnée dans le tableau II a été utilisé en mélange avec de l'isomaltulose cristallisé commercialisé par la Société MITSUI pour préparer des mélanges renfermant respectivement :
. 35 % d'isomaltulose (sur base sèche)
. 50 % d'isomaltulose (sur base sèche)
. 66 % d'isomaltulose (sur base sèche)
et ces sirops ont été hydrogénés, purifiés et concentrés pour obtenir les compositions E, F et G selon l'invention et possédant les spectres glucidiques (exprimés en % sur matière sèche) repris dans le tableau IV.

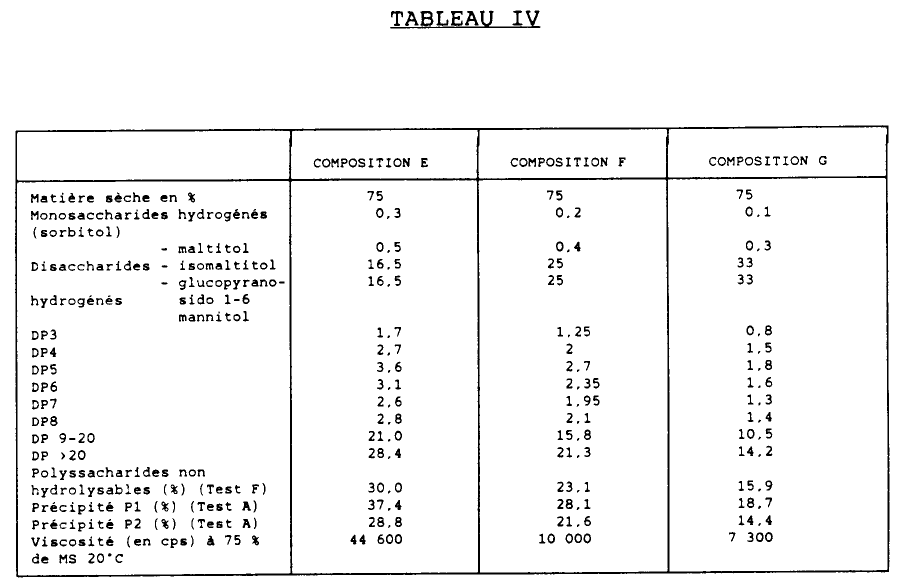

### EXEMPLE N° 4

Dans une cuve de 25 litres agitée et thermostatée, on introduit 20 litres d'un sirop formé par dilution dans l'eau, à une matière sèche de 35 %, de dextrine jaune TACKIDEX DF 165 commercialisée par la Société Demanderesse. On ajuste le pH de ce sirop à 5,5 et la température à 55°C, puis on introduit :
- 1,5 °/°° (p/p sec) de β-amylase Spezyme DBA de la Société GENENCOR et 2 °/°° (p/p sec) de pullulanase. Pulluzyme 750 L de la Société ABM.

Après 86 heures, on acidifie à pH 3,5 et on chauffe la cuve à 80°C pendant 20 minutes pour inhiber les enzymes.

Ce sirop est filtré, puis déminéralisé sur résines cationique forte et anionique faible et amené à une matière sèche de 40 %.

Cet hydrolysat est ensuite hydrogéné. Pour cela, on ajoute par rapport au sirop 5 % de catalyseur au nickel de Raney. L'hydrogénation est conduite à une température de 130°C sous une pression d'hydrogène de 50 bars. Elle est poursuivie jusqu'à l'obtention d'un taux de sucres réducteurs inférieur à 0,5 %.

L'hydrolysat hydrogéné est ensuite purifié et concentré à 70 % de matière sèche. Il contient environ, par rapport au poids sec :
- 1 % de sorbitol,
- 22 % de maltitol et d'isomaltitol,
- 25 % de polysaccharides non hydrolysables selon le test F.

Ce sirop conforme à l'invention, appelé par la suite sirop hydrogéné S a servi de base pour confectionner les compositions H, I et J selon l'invention.

### Composition H

Une partie du sirop hydrogéné S est dilué à 60 % de matière sèche.

Du mannitol F commercialisé par la Société Demanderesse est ajouté à ce sirop hydrogéné S dilué, en proportion telle que le mannitol représente 25 % de la matière sèche de la composition totale. Le mélange est agité jusqu'à dissolution complète du mannitol, puis concentré à 70 % de matière sèche pour constituer la composition H.

### Composition I

Dans une cuve de 25 litres agitée et thermostatée à 60°C, on introduit :
- 4 kg de sirop hydrogéné S,
- 16 kg de sirop de sorbitol NEOSORB ^{R} 70/70 commercialisé par la Société Demanderesse.

On a obtenu ainsi, après mélange intime, la composition I.

### Composition J

Da1ns une cuve de 25 litres agitée et thermostatée à 65°C, on a introduit :
- 10 kg de sirop hydrogéné S,
- 10 kg de sirop de polyols POLYSORB ^{R} 70/12/12 commercialisé par la Demanderesse.

Ces compositions ont révélé les spectres glucidiques suivants (tableau V)

**TABLEAU V**

| | Composition H | Composition I | Composition J |
|---|---|---|---|
| Matière sèche en % | 70 | 70 | 70 |
| Monosaccharides en % | | | |
| Sorbitol | 0,8 | 60,3 | 8,0 |
| Mannitol | 25,2 | 5,8 | 0,5 |
| Disaccharides en % | 16,5 | 14,3 | 16,9 |
| Polysaccharides non hydrolysables en % ( Test F) | 16,9 | 4,6 | 11,4 |

Les compositions H et I sont non cariogènes selon le test B. La composition J peut être considérée comme hypocariogène.

La composition H s'est révélée très intéressante pour préparer des sucres cuits sans sucre peu hygroscopiques.

La composition I a donné de bons résultats dans la fabrication de dentifrices, conférant à ces derniers associés aux silices une texture gélifiée remarquable.

Enfin, la composition J a été utilisée dans la préparation de moules de fonderie silicatés. Il s'agit là d'un bon agent de liaison et de cohésion.

### EXEMPLE N° 5 : Préparation de pâtes à mâcher non cariogènes comprenant quatre compositions selon l'invention.

Les compositions A, B, C et D obtenues selon les exemples n° 1 et 2 sont utilisées pour formuler des pâtes à mâcher non cariogènes.

Une pâte à mâcher témoin est préparée en utilisant un mélange de xylitol cristallisé et de sirop LYCASIN ^{R} 80/55. Les quatre recettes présentent ainsi des teneurs voisines en xylitol.

Les recettes (en grammes) avant cuisson des pâtes à mâcher sont données dans le tableau VI.

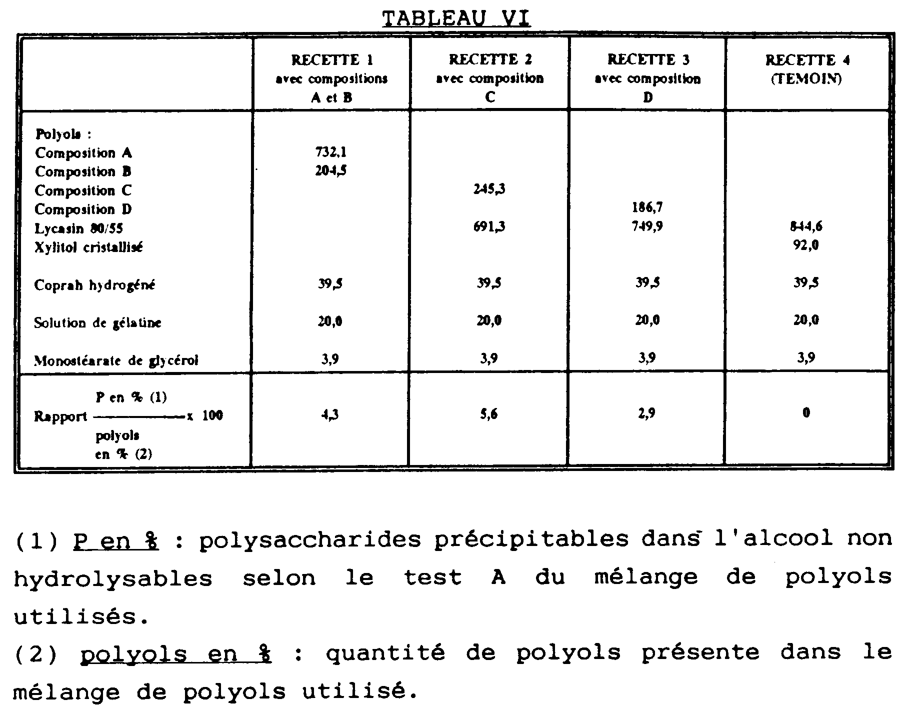

Pour fabriquer les pâtes à mâcher, on procède à une cuisson des mélanges de polyols à une température choisie (130°C ou 145°C), à pression atmosphérique, on laisse refroidir vers 110°C, on incorpore la matière grasse, l'émulsifiant et la solution de gélatine, puis on étire les pâtes obtenues avant de les mettre en forme et de les découper.

Les caractéristiques rhéologiques des pâtes à mâcher obtenues sont données dans le tableau VII ci-dessous :

**TABLEAU VII**

| TEMPERATURE DE CUISSON C° | RECETTE 1 | RECETTE 2 | RECETTE 3 | RECETTE 4 |
|---|---|---|---|---|
| | Pâte : | Pâte : | Pâte : | Pâte : |
| 130°C | . de texture correcte | . molle | . de texture correcte | . très molle |
| | . peu collante | . légèrement collante | . très légèrement collante | . très collante |
| | . bonne machinabilité | . difficile à former | . bonne machinabilité | . impossible à former |
| | . tenue correcte | . tenue incorrecte | . tenue correcte | . pas de tenue |

| | Pâte : | Pâte : | Pâte : | Pâte : |
|---|---|---|---|---|
| 145°C | . de texture ferme | . de texture plutôt cassante | . de texture correcte | . molle |
| | . non collante | . non collante | . très légèrement collante | . très collante |
| | . bonne machinabilité | . bonne machinabilité | . bonne machinabilité | . difficile à former |
| | . tenue correcte | . tenue correcte | . tenue correcte | . tenue correcte |

On peut constater que par rapport aux possibilités offertes dans l'art antérieur, les compositions A, B, C et D permettent de préparer des pâtes à mâcher non cariogènes contenant du xylitol, de texture convenant à un travail sur machine et ayant une tenue correcte au stockage.

### EXEMPLE N° 6

Préparation de bonbons au sucre cuit hypocariogèénes, comprenant des compositions selon l'invention.

Les compositions F et G obtenues selon l'exemple 3 sont utilisées pour préparer des bonbons au sucre cuit. Pour cela, ces deux compositions d'une matière sèche de 75 % sont déshydratées par cuisson à feu nu et à pression atmosphérique aux températures de 140°C, 145 °C, 150°C, 160°C.

Des bonbons témoins au sucre cuit sont obtenus par cuisson d'ISOMALT ^{R} (mélange équimoléculaire d'isomaltitol et de glucopyranosido 1-6 mannitol) aux mêmes températures.

Après cuisson, on obtient des bonbons dont les teneurs en eau s'avèrent être les suivantes :

**TABLEAU VIII**

| TEMPERATURE DE CUISSON | COMPOSITION F | COMPOSITION G | ISOMALT |
|---|---|---|---|
| | EAU % | EAU % | EAU % |
| 140°C | 3,6 | 4,0 | 6,5 |
| 145°C | 2,5 | 3,1 | 5,0 |
| 150°C | 2,1 | 2,4 | 4,5 |
| 160°C | 1,3 | 2,0 | 3,1 |

On constate que la déshydratation des compositions F et G selon l'invention est plus aisée que celle du sirop d'ISOMALT. La cuisson des compositions selon l'invention requiert moins d'énergie, et elles permettent d'obtenir des bonbons présentant, tout comme les bonbons traditionnels obtenus avec le sucre, une teneur en eau comprise entre 2 et 3 % nécessaire pour conférer une bonne stabilité aux bonbons, malgré des températures de cuisson peu élevées. Les bonbons obtenus sont de surcroit hypocariogènes.

## Revendications

1. Composition de saccharides hydrogénés hypocariogènes caractérisée par le fait qu'elle présente, les teneurs étant exprimées en poids par rapport à la matière sèche de la composition :
- une teneur de 0,1 à 80 % en monosaccharides hydrogénés,
- une teneur de 0,1 à 96 % en disaccharides hydrogénés,
- une teneur de 11 à 96 % en mono et en disaccharides hydrogénés,
- une teneur de 1 à 40 % en polysaccharides non hydrolysables par l'amyloglucosidase dans un test F tel que défini dans la description,
le complément à 100 % étant constitué par des oligosaccharides et des polysaccharides hydrogénés.

2. Composition selon la revendication 1, caractérisée par le fait que la teneur en mono et disaccharides hydrogénés est de 22 à 94 %.

3. Composition selon la revendication 2, caractérisée par le fait que la teneur en mono et disaccharides hydrogénés est de 35 à 90 %.

4. Composition selon l'une quelconque des revendications 1 à 3, caractérisée par le fait que la teneur en monosaccharides hydrogénés est de 0,1 à 75 %.

5. Composition selon la revendication 4, caractérisée par le fait que la teneur en monosaccharides hydrogénés est de 0,1 à 70 %.

6. Composition selon l'une quelconque des revendications 1 à 5, caractérisée par le fait que la teneur en disaccharides hydrogénés est de 0,2 à 94 %.

7. Composition selon la revendication 6, caractérisée par le fait que la teneur en disaccharides hydrogénés est de 0,3 à 90 %.

8. Composition selon l'une quelconque des revendications 1 à 7, caractérisée par le fait que la teneur en polysaccharides non hydrolysables par l'amyloglucosidase dans le test F est de 1,5 à 30 %.

9. Composition selon la revendication 8, caractérisée par le fait que la teneur en polysaccharides non hydrolysables par l'amyloglucosidase dans le test F est de 3 à 26,5 %.

10. Composition de saccharides hydrogénés hypocariogènes caractérisée par le fait qu'elle présente, les teneurs étant exprimées en poids par rapport à la matière sèche de la composition :
- une teneur de 0,1 à 65 % en monosaccharides hydrogénés,
- une teneur de 10 à 96 % en disaccharides hydrogénés,
- une teneur de 1 à 30 % en polysaccharides précipitables dans l'éthanol et non hydrolysables par l'amyloglucosidase dans un test A tel que défini dans la description,
le complément à 100 % étant constitué par des oligosaccharides et des polysaccharides hydrogénés.

11. Composition selon la revendication 10, caractérisée en ce que la teneur en polysaccharides précipitables dans l'éthanol et non hydrolysables dans le test A est de 1,5 à 25 %.

12. Composition selon la revendication 11, caractérisée par le fait que la teneur en polysaccharides précipitables dans l'éthanol et non hydrolysables par l'amyloglucosidase dans le test A est de 2 à 15 %.

13. Composition selon l'une quelconque des revendications 1 à 12, caractérisée par le fait que les monosaccharides hydrogénés sont choisis parmi le sorbitol, le mannitol, le galactitol, le xylitol, le thréitol, l'arabitol et l'érythritol.

14. Composition selon l'une quelconque des revendications 1 à 13, caractérisée par le fait que les disaccharides hydrogénés sont choisis parmi le maltitol, le maltulose hydrogéné, l'isomaltulose hydrogéné, l'isomaltitol, le lactitol, l'inulobiose hydrogéné.

15. Composition selon l'une quelconque des revendications 1 à 14, caractérisée par le fait que les oligosaccharides et polysaccharides hydrogénés sont choisis parmi le maltotriitol, le maltotétraitol, l'inulotriitol, les oligosaccharides et polysaccharides hydrogénés obtenus par hydrolyse de l'amidon suivi d'une hydrogénation, le cellobiitol, le cellotriitol, le xylobiitol, le xylotriitol et les oligosaccharides et polysaccharides hydrogénés obtenus par hydrolyse de cellulose, de xylans, ou de fructans, puis hydrogénation.

16. Composition selon l'une quelconque des revendications 1 à 15, caractérisée par le fait qu'elle n'est pas cariogène selon le test B tel que défini dans la description.

17. Composition selon l'une quelconque des revendications 1 à 16, caractérisée par le fait que les polysaccharides non hydrolysables par l'amyloglucosidase sont obtenus par traitement enzymatique d'une dextrine et/ou d'un polyglucose, ce traitement comportant au moins l'action d'une enzyme saccharifiante telle que l'amyloglucosidase ou la béta-amylase, le produit saccharifié ainsi obtenu étant ensuite hydrogéné.

18. Procédé de préparation d'une composition de saccharides hydrogénés hypocariogènes selon l'une quelconque des revendications 1 à 17, caractérisé par le fait que l'on prépare tout d'abord une fraction comprenant des polysaccharides non hydrolysables par l'amyloglucosidase en soumettant au moins une dextrine ou un polyglucose à un traitement enzymatique comportant au moins l'action d'une enzyme saccharifiante telle que l'amyloglucosidase ou la béta-amylase, les conditions de ce traitement étant choisies de façon telle que le DE de l'hydrolysat obtenu soit compris entre 5 et 80 à l'issue de ce traitement, que l'on ajoute à cette fraction des monosaccharides, des disaccharides ou des oligosaccharides et polysaccharides dans des proportions choisies en chacun de ces différents constituants, et que l'on réalise l'hydrogénation de la composition ainsi obtenue.

19. Procédé de préparation d'une composition de saccharides hydrogénés hypocariogènes selon l'une quelconque des revendications 1 à 17, caractérisé parle fait que l'on prépare tout d'abord une fraction comprenant des polysaccharides non digestibles à l'amyloglucosidase en soumettant au moins une dextrine et/ou un polyglucose à un traitement enzymatique comportant au moins l'action d'une enzyme saccharifiante telle que l'amyloglucosidase ou la béta-amylase, les conditions de ce traitement étant choisies de façon telle que le DE de l'hydrolysat obtenu soit compris entre 5 et 80 à l'issue de ce traitement, que l'on hydrogène cette fraction, et que l'on ajoute à la fraction hydrogénée ainsi obtenue des monosaccharides, des disaccharides, des oligosaccharides et polysaccharides hydrogénés dans des proportions choisies en chacun de ces différents constituants.

20. Procédé selon l'une ou l'autre des revendications 18 et 19, caractérisé par le fait que le traitement enzymatique comportant au moins l'action d'une enzyme saccharifiante est précédé, accompagné, ou suivi d'un traitement à l'aide d'une alpha-amylase.

21. Procédé selon l'une quelconque des revendications 18 à 20, caractérisé par le fait que le traitement enzymatique comportant au moins l'action d'une enzyme saccharifiante est précédé, accompagné, ou suivi d'un traitement à l'aide d'une enzyme hydrolysant les liaisons 1-6 de l'amylopectine.

22. Application de la composition de saccharides hydrogénés hypocariogènes selon l'une quelconque des revendications 1 à 17 comme composition édulcorante ou comme agent de texture dans les produits destinés à être ingérés par les hommes ou les animaux.

## Claims

1. Composition containing hypocariogenic hydrogenated saccharides, characterised in that it has, the concentrations being expressed by weight relative to the dry matter of the composition:
- a concentration of 0.1 to 80% of hydrogenated monosaccharides,
- a concentration of 0.1 to 96% of hydrogenated disaccharides,
- a concentration of 11 to 96% of hydrogenated mono-and disaccharides,
- a concentration of 1 to 40% of polysaccharides which are not hydrolised by amyloglucosidase in an F test such as defined in the specification,
the balance for 100% consisting of hydrogenated oligo- and polysaccharides.

2. Composition according to Claim 1, characterised in that the concentration of hydrogenated mono- and disaccharides is of 22 to 94%.

3. Composition according to Claim 2, characterised in that the concentration of hydrogenated mono- and disaccharides is of 35 to 90%.

4. Composition according to any one of Claims 1 to 3, characterised in that the concentration of hydrogenated monosaccharides is of 0.1 to 75%.

5. Composition according to Claim 4, characterised in that the concentration of hydrogenated monosaccharides is of 0.1 to 70%.

6. Composition according to any one of Claims 1 to 5, characterised in that the concentration of hydrogenated disaccharides is of 0.2 to 94%.

7. Composition according to Claim 6, characterised in that the concentration of hydrogenated disaccharides is of 0.3 to 90%.

8. Composition according to any one of Claims 1 to 7, characterised in that the concentration of polysaccharides which are not hydrolysed by amyloglucosidase in the F test is of 1.5 to 30%.

9. Composition according to Claim 8, characterised in that the concentration of polysaccharides which are not hydrolysed by amyloglucosidase in the F test is of 3 to 26.5%.

10. Composition containing hypocariogenic hydrogenated saccharides, characterised in that it has, the concentrations being expressed by weight relative to the dry matter of the composition:
- a concentration of 0.1 to 65% of hydrogenated monosaccharides,
- a concentration of 10 to 96% of hydrogenated disaccharides,
- a concentration of 1 to 30% of polysaccharides which can be precipitated in ethanol and which are not hydrolysed by amyloglucosidase in an A test such as defined in the specification,
the balance for 100% consisting of hydrogenated oligosaccharides or polysaccharides.

11. Composition according to Claim 10, characterised in that the concentration of polysaccharides which can be precipitated in ethanol and which are not hydrolysed in the A test is of 1.5 to 25%.

12. Composition according to Claim 11, characterised in that the concentration of polysaccharides which can be precipitated in ethanol and which are not hydrolysed by amyloglucosidase in the A test is of 2 to 15%.

13. Composition according to any one of Claims 1 to 12, characterised in that the hydrogenated monosaccharides are chosen from the group comprising sorbitol, mannitol, galactitol, xylitol, threitol, arabitol and erythrithol.

14. Composition according to any one of Claims 1 to 13, characterised in that the hydrogenated disaccharides are chosen from the group comprising maltitol, hydrogenated maltulose, hydrogenated isomaltulose, isomaltitol, lactitol, and hydrogenated inulobiose.

15. Composition according to any one of Claims 1 to 14, characterised in that the hydrogenated oligosaccharides and polysaccharides are chosen from the group comprising maltotriitol, maltotetraitol, inulotriitol, the hydrogenated oligosaccharides and polysaccharides obtained by hydrolysis of starch followed by hydrogenation, cellobiitol, cellotriitol, xylobiitol, xylotriitol and the hydrogenated oligosaccharides and polysaccharides obtained by hydrolysis of cellulose, xylans, or fructans, followed by hydrogenation.

16. Composition according to any one of Claims 1 to 15, characterised in that it is not cariogenic based on the B test such as defined in the specification.

17. Composition according to any one of Claims 1 to 16, characterised in that the polysaccharides not hydrolysed by amyloglucosidase are obtained by enzymatic treatment of a dextrin and/or a polyglucose, this treatment comprising at least the action of a saccharifying enzyme such as amyloglucosidase or beta-amylase, the saccharified product thus obtained then being hydrogenated.

18. Process for preparing a composition containing hypocariogenic hydrogenated saccharides according to any one of Claims 1 to 17, characterised in that there is first prepared a fraction comprising polysaccharides not hydrolysed by amyloglucosidase, by subjecting a dextrin or a polyglucose to an enzymatic treatment comprising at least the action of a saccharifying enzyme such as amyloglucosidase or beta-amylase, the conditions for this treatment being chosen such that the DE of the hydrolysate obtained is between 5 and 80 at the end of this treatment, in that there are added to this fraction monosaccharides, disaccharides or oligosaccharides and polysaccharides in chosen proportions for each of these various constituents, and in that the composition thus obtained is hydrogenated.

19. Process for preparing a composition containing hypocariogenic hydrogenated saccharides according to any one of Claims 1 to 17, characterised in that there is first prepared a fraction comprising polysaccharides not digested by amyloglucosidase, by subjecting at least one dextrin and/or one polyglucose to an enzymatic treatment comprising at least the action of a saccharifying enzyme such as amyloglucosidase or beta-amylase, the conditions for this treatment being chosen such that the DE of the hydrolysate obtained is between 5 and 80 at the end of this treatment, in that this fraction is hydrogenated, and in that there are added to the hydrogenated fraction thus obtained hydrogenated monosaccharides, disaccharides, oligosaccharides and polysaccharides in chosen proportions for each of these various constituents.

20. Process according to either of Claims 18 and 19, characterised in that the enzymatic treatment comprising at least the action of a saccharifying enzyme is preceded, accompanied or followed by a treatment using an alpha-amylase.

21. Process according to any one of Claims 18 to 20, characterised in that the enzymatic treatment comprising at least the action of a saccharifying enzyme is preceded, accompanied or followed by a treatment using an enzyme which hydrolyses the 1-6 bonds of amylopectin.

22. Application of the composition containing hypocariogenic hydrogenated saccharides according to any one of Claims 1 to 17 as sweetening composition or as texturing agent in products intended to be ingested by humans or animals.

## Patentansprüche

1. Hypocariogene hydrierte Saccharidzusammensetzung, dadurch **gekennzeichnet,** daß sie ausgedrückt als Gewichtsgehalte bezogen auf die Trockenmasse der Zusammensetzung:
- einen Gehalt von 0,1 bis 80 % hydrierte Monosaccharide,
- einen Gehalt von 0,1 bis 96 % hydrierte Disaccharide,
- einen Gehalt von 11 bis 96 % hydrierte Mono- und Disaccharide,
- einen Gehalt von 1 bis 40 % durch Amyloglucosidase in einem F-Test, wie in der Beschreibung definiert, nicht hydrolysierbare Polysaccharide enthält,
wobei der Rest zu 100 % aus hydrierten Oligosacchariden und Polysacchariden besteht.

2. Zusammensetzung nach Anspruch 1, dadurch **gekennzeichnet,** daß der Gehalt an hydrierten Mono- und Disacchariden 22 bis 94 % beträgt.

3. Zusammensetzung nach Anspruch 2, dadurch **gekennzeichnet,** daß der Gehalt an hydrierten Mono- und Disacchariden 35 bis 90 % beträgt.

4. Zusammensetzung nach einem der Ansprüche 1 bis 3, dadurch **gekennzeichnet,** daß der Gehalt an hydrierten Monosacchariden 0,1 bis 75 % beträgt.

5. Zusammensetzung nach Anspruch 4, dadurch **gekennzeichnet,** daß der Gehalt an hydrierten Monosacchariden 0,1 bis 70 % beträgt.

6. Zusammensetzung nach einem der Ansprüche 1 bis 5, dadurch **gekennzeichnet,** daß der Gehalt an hydrierten Disacchariden 0,2 bis 94 % beträgt.

7. Zusammensetzung nach Anspruch 6, dadurch **gekennzeichnet,** daß der Gehalt an hydrierten Disaccharlden 0,3 bis 90 % beträgt.

8. Zusammensetzung nach einem der Ansprüche 1 bis 7, dadurch **gekennzeichnet,** daß der Gehalt an durch Amyloglucosidase in dem F-Test nicht hydrolysierbaren Polysacchariden 1,5 bis 30 % beträgt.

9. Zusammensetzung nach Anspruch 8, dadurch **gekennzeichnet,** daß der Gehalt an durch Amyloglucosidase in dem F-Test nicht hydrolysierbaren Polysacchariden 3 bis 26,5 % beträgt.

10. Hypocariogene hydrierte Saccharidzusammensetzung, dadurch **gekennzeichnet,** daß sie ausgedrückt als Gewichtsgehalte bezogen auf die Trockenmasse der Zusammensetzung:
- einen Gehalt von 0,1 bis 65 % hydrierte Monosaccharide,
- einen Gehalt von 10 bis 96 % hydrierte Disaccharide,
- einen Gehalt von 1 bis 30 % in Ethanol ausfällbare und in einem A-Test, wie in der Beschreibung definiert, durch Amyloglucosidase nicht hydrolysierbare Polysaccharide enthält, wobei der Rest zu 100 % aus hydrierten Oligosacchariden und Polysacchariden besteht.

11. Zusammensetzung nach Anspruch 10, dadurch **gekennzeichnet,** daß der Gehalt an in Ethanol ausfällbaren und in dem A-Test nicht hydrolysierbaren Polysacchariden 1,5 bis 25 % beträgt.

12. Zusammensetzung nach Anspruch 11, dadurch **gekennzeichnet,** daß der Gehalt an in Ethanol ausfällbaren und in dem A-Test nicht hydrolysierbaren Polysacchariden 2 bis 15 % beträgt.

13. Zusammensetzung nach einem der Ansprüche 1 bis 12, dadurch **gekennzeichnet,** daß die hydrierten Monosaccharide ausgewählt sind aus Sorbit, Mannit, Galactit, Xylit, Threit, Arabit und Erythrit.

14. Zusammensetzung nach einem der Ansprüche 1 bis 13, dadurch **gekennzeichnet,** daß die hydrierten Disaccharide ausgewählt sind aus Maltit, hydrierter Maltulose, hydrierter Isomaltulose, Isomaltit, Lactit, hydrierter Inulobiose.

15. Zusammensetzung nach einem der Ansprüche 1 bis 14, dadurch **gekennzeichnet,** daß die hydrierten oligosaccharide und Polysaccharide ausgewählt sind aus Maltotriit, Maltotetrait, Inulotriit, hydrierten Oligosacchariden und Polysacchariden, erhalten durch Hydrolyse von Stärke, gefolgt von einer Hydrierung, Cellobiit, Cellotriit, Xylobiit, Xylotriit und hydrierten Oligosacchariden und Polysacchariden, erhalten durch Hydrolyse von Cellulose, Xylanen oder Fructanen mit anschließender Hydrierung.

16. Zusammensetzung nach einem der Ansprüche 1 bis 15, dadurch **gekennzeichnet,** daß sie in dem B-Test, wie in der Beschreibung definiert, nicht cariogen ist.

17. Zusammensetzung nach einem der Ansprüche 1 bis 16, dadurch **gekennzeichnet,** daß die durch Amyloglycosidase nicht hydrolysierbaren Polysaccharide durch enzymatische Behandlung eines Dextrins und/oder einer Polyglucose erhalten werden, wobei diese Behandlung mindestens die Wirkung eines saccharidspaltenden Enzyms, wie Amyloglucosidase oder β-Amylase, umfaßt, wobei das so erhaltene in ein Saccharid überführte Produkt anschließend hydriert wird.

18. Verfahren zur Herstellung einer hypocariogenen hydrierten Saccharidzusammensetzung nach einem der Ansprüche 1 bis 17, dadurch **gekennzeichnet,** daß man zuerst eine Fraktion aus durch Amyloglucosidase nicht hydrolysierbaren Polysacchariden herstellt, indem man mindestens ein Dextrin oder eine Polyglucose einer enzymatischen Behandlung mit mindestens der Wirkung eines saccharidspaltenden Enzyms, wie Amyloglucosidase oder β-Amylase, unterwirft, wobei die Bedingungen dieser Behandlung so ausgewählt werden, daß der DE-Wert des so erhaltenen Hydrolysats am Ende dieser Behandlung zwischen 5 und 80 liegt, dann dieser Fraktion Monosaccharide, Disaccharide oder Oligosaccharide und Polysaccharide in den gewählten Verhältnissen an jedem dieser verschiedenen Bestandteile zusetzt und anschließend die so erhaltene Zusammensetzung hydriert.

19. Verfahren zur Herstellung einer hypocariogenen hydrierten Saccharidzusammensetzung nach einem der Ansprüche 1 bis 17, dadurch **gekennzeichnet,** daß man zuerst eine Fraktion aus durch Amyloglucosidase nicht spaltbaren Polysacchariden herstellt, indem man mindestens ein Dextrin und/oder eine Polyglucose einer enzymatischen Behandlung mit mindestens der Wirkung eines saccharidspaltenden Enzyms, wie Amyloglucosidase oder β-Amylase, unterwirft, wobei die Bedingungen dieser Behandlung so ausgewählt sind, daß der DE-Wert des so erhaltenen Hydrolysats am Ende dieser Behandlung zwischen 5 und 80 liegt, dann diese Fraktion hydriert und der so erhaltenen hydrierten Fraktion hydrierte Monosaccharide, Disaccharide, Oligosaccharide und Polysaccharide in den für jeden dieser verschiedenen Bestandteile gewählten Verhältnissen zusetzt.

20. Verfahren nach einem der Ansprüche 18 und 19, dadurch **gekennzeichnet,** daß der enzymatischen Behandlung, umfassend mindestens die Wirkung eines saccharidspaltenden Enzyms, eine Behandlung mit einer α-Amylase vorausgeht, sie begleitet oder auf sie folgt.

21. Verfahren nach einem der Ansprüche 18 bis 20, dadurch **gekennzeichnet,** daß der enzymatischen Behandlung, umfassend mindestens die Wirkung eines saccharidspaltenden Enzyms, eine Behandlung mit einem Enzym, das 1-6-Bindungen von Amylopectin hydrolysiert, vorausgeht, sie begleitet oder auf sie folgt.

22. Verwendung der hypocariogenen hydrierten Saccharidzusammensetzung nach einem der Ansprüche 1 bis 17 als Süßstoff oder als Texturstoff in Produkten, die für den menschlichen oder tierischen Verzehr bestimmt sind.
